(19) **Europäisches Patentamt** / **European Patent Office** / **Office européen des brevets**

(11) **EP 4 730 234 A1**

(12) **EUROPEAN PATENT APPLICATION**
published in accordance with Art. 153(4) EPC

(43) Date of publication:
**22.04.2026 Bulletin 2026/17**

(21) Application number: **23941651.4**

(22) Date of filing: **16.06.2023**

(51) International Patent Classification (IPC):
***G06Q 10/0633*** (2023.01)

(52) Cooperative Patent Classification (CPC):
**G06Q 10/0633**

(86) International application number:
**PCT/JP2023/022487**

(87) International publication number:
**WO 2024/257352 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Fujitsu Limited
Kawasaki-shi, Kanagawa 211-8588 (JP)**

(72) Inventors:
- **SASAMOTO, Yuki
  Kawasaki-shi, Kanagawa 211-8588 (JP)**
- **SAWASAKI, Naoyuki
  Kawasaki-shi, Kanagawa 211-8588 (JP)**
- **KURAKI, Kensuke
  Kawasaki-shi, Kanagawa 211-8588 (JP)**
- **INOMATA, Akihiro
  Kawasaki-shi, Kanagawa 211-8588 (JP)**

(74) Representative: **Hoffmann Eitle
Patent- und Rechtsanwälte PartmbB
Arabellastraße 30
81925 München (DE)**

(54) **GENERATION METHOD, GENERATION PROGRAM, AND INFORMATION PROCESSING DEVICE**

(57)    In a generation method, a computer executes: acquiring a first measure and a second measure configured from graph data including a plurality of conditional branches coupled by a directed edge and nodes coupled to destinations to which each of the plurality of conditional branches is branched; generating information regarding a first difference between the acquired first and second measures, the first difference being at least one of a difference in structure of graph data and a difference in branch probability of an object assigned to a node of the conditional branch; and generating correspondence information in which the generated information regarding the first difference is associated with information regarding a second difference between the acquired first and second measures, the second difference being at least one of a difference in effect value and a difference in certainty factor.

FIG.4

## Description

Technical Field

The present invention relates to a generation method, a generation program, and an information processing apparatus.

Background Art

As one type of workflow, a flow graph for a measure is known in which a flow of assigning an object that is a target of the measure in various fields such as medical care, nursing care, and administration, for example, a human, to a service for achieving the purpose of the measure or the like is schematized.

For example, various technologies for predicting effects of measures have been proposed to support the planning of measures. One aspect of using such technologies is to plan a measure by calculating effects of a plurality of measures and selecting the most effective measure from among the plurality of measures.

Citation List

Patent Literature

Patent Document 1: Japanese Laid-open Patent Publication No. 2021-117837

Summary of invention

Technical Problem

However, even if the most effective measure is selected, it is not always easy to change to the selected measure. For example, in order to implement the most effective measure, resources corresponding to that effect are required. However, the current resources may be insufficient to meet the required amount, it may be difficult to increase the resources, or the cost may be unacceptable even if the resources can be increased.

In one aspect, an object of the present invention is to provide a generation method, a generation program, and an information processing apparatus capable of evaluating an influence of a measure change.

Solution to Problem

According to an aspect of the embodiment of the invention, a generation method is executed by a computer, the generation method includes: acquiring a first measure and a second measure configured from graph data including a plurality of conditional branches coupled by a directed edge and nodes coupled to destinations to which each of the plurality of conditional branches is branched; generating information regarding a first difference between the acquired first and second measures, the first difference being at least one of a difference in structure of graph data and a difference in branch probability of an object assigned to a node of the conditional branch; and generating correspondence information in which the generated information regarding the first difference is associated with information regarding a second difference between the acquired first and second measures, the second difference being at least one of a difference in effect value and a difference in certainty factor.

Advantageous Effects of Invention

According to one embodiment, it is possible to evaluate an influence of a measure change.

Brief Description of Drawings

FIG. 1 is a block diagram illustrating an example of a functional configuration of a server device.
FIG. 2 is a diagram illustrating an example of a flow graph for a measure.
FIG. 3 is a diagram illustrating a specific example of a flow graph for a measure.
FIG. 4 is a diagram illustrating one aspect of a problem solving approach.
FIG. 5 is a diagram (1) illustrating an example in which evaluation values are displayed.

FIG. 6 is a diagram (2) illustrating an example in which evaluation values are displayed.
FIG. 7 is a schematic diagram (1) for explaining an example of a numerical calculation of an area change evaluation value.
FIG. 8 is a schematic diagram (2) for explaining an example of a numerical calculation of an area change evaluation value.
FIG. 9 is a diagram (3) illustrating an example in which evaluation values are displayed.
FIG. 10 is a diagram (4) illustrating an example in which evaluation values are displayed.
FIG. 11 is a flowchart illustrating calculation processing steps.
FIG. 12 is a diagram illustrating an example of a hardware configuration.

Description of Embodiments

Hereinafter, a generation method, a generation program, and an information processing apparatus according to embodiments of the present application will be described with reference to the accompanying drawings. Each embodiment merely represents an example or an aspect, and a numerical value, a functional range, a usage scene, and the like are not limited by such an example. Then, the embodiments can be appropriately combined within a range in which the processing contents do not contradict each other.

<First Embodiment>

<System Configuration>

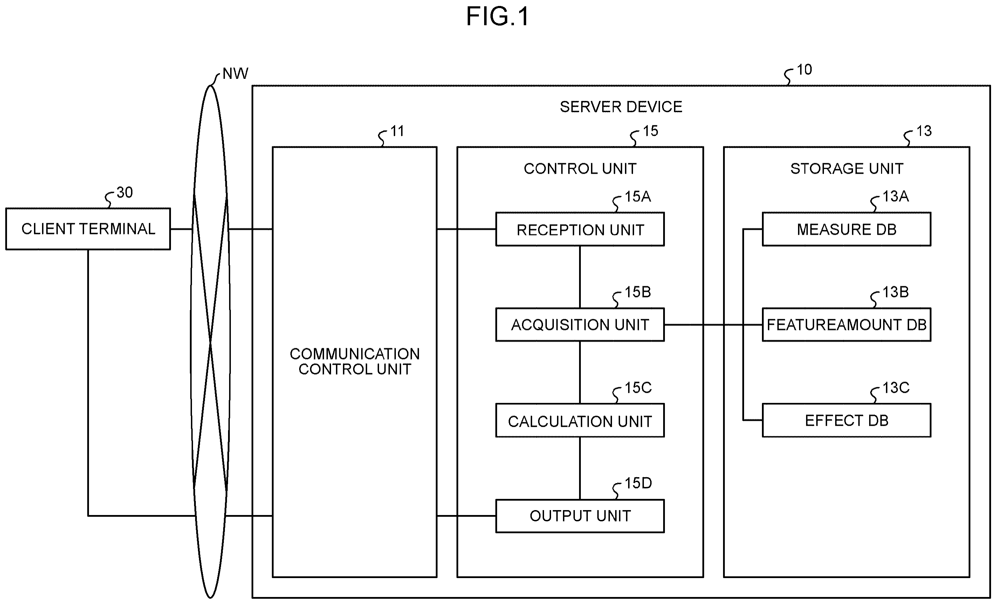

FIG. 1 is a block diagram illustrating an example of a functional configuration of a server device 10. The server device 10 illustrated in FIG. 1 provides a data infrastructure platform capable of sharing, cross-referencing, and updating flow graphs for measures.
For example, the server device 10 can provide the functions of the above-described data infrastructure platform as a cloud service by executing platform as a service (PaaS) type middleware or software as a service (SaaS) type applications. Note that the server device 10 corresponds to an example of an information processing apparatus.
As illustrated in FIG. 1, the server device 10 can be communicably connected to a client terminal 30 via a network NW. For example, the network NW may be any type of communication network, such as the Internet or a local area network (LAN), regardless of whether it is wired or wireless. Note that FIG. 1 illustrates an example in which one client terminal 30 is connected to one server device 10, but any number of client terminals 30 may be connected to one server device 10.
The client terminal 30 is a terminal device that is provided with the above-mentioned data infrastructure. For example, the client terminal 30 can be used by a measure planner as an example of a person involved in an entity implementing a measure, for example, a local government. Note that, as an example, the client terminal 30 may be realized by any computer such as not only a personal computer but also a smartphone, a tablet terminal, or a wearable terminal.

<Flow Graph for Measure>

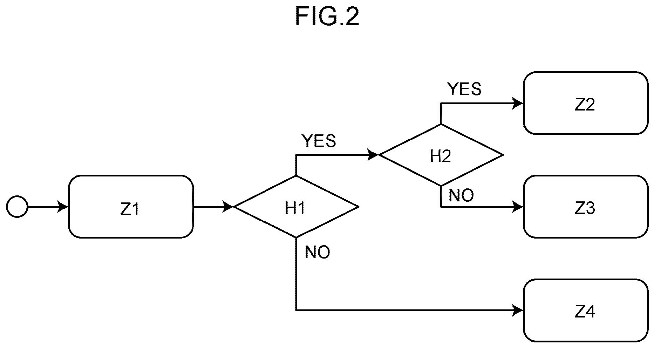

An example of a flow graph for the above-described measure is illustrated in FIG. 2. FIG. 2 is a diagram illustrating an example of a flow graph for a measure. Z1, Z2, Z3, and Z4 illustrated in FIG. 2 indicate, for example, services provided by an administrator to a user. In addition, these may be referred to as "service implementation components". Specific examples of the services include, for example, in the medical field, "intervention", such as a medical examination through a health checkup and an examination by a specialist, to which an object who is a target of the measure, such as a resident, is assigned, as well as "no intervention" such as follow-up observation, but are not limited to measures in the medical field.
H1 and H2 indicate, for example, conditional branches including conditions. In addition, these may be referred to as "conditional branch components". Specific examples of the conditions include, for example, in the medical field, an estimated glemerular filtration rate (eGFR) smaller than a threshold value, a hemoglobin A1c value (HbA1c) smaller than a threshold value, and a urinary protein value equal to or larger than a threshold value, but are not limited to conditions in the medical field.
Each of Z1, Z2, Z3, Z4, H1, and H2 may be referred to as a "component". Such a "component" may correspond to an example of a "node" in terms of graph data. Hereinafter, a node corresponding to a branch among the nodes may be referred to as a "branch node". Furthermore, a connection between nodes may correspond to an example of an "edge" including a "directed edge" and the like.
Note that, in the present embodiment, measure planning in the medical field will be described as an example, but the present invention is not limited thereto. The above-described embodiment may be used for planning various measures such as tasks, tests, and questionnaires having conditional branches. In this case as well, the same effects as those of the above-described embodiment can be obtained.

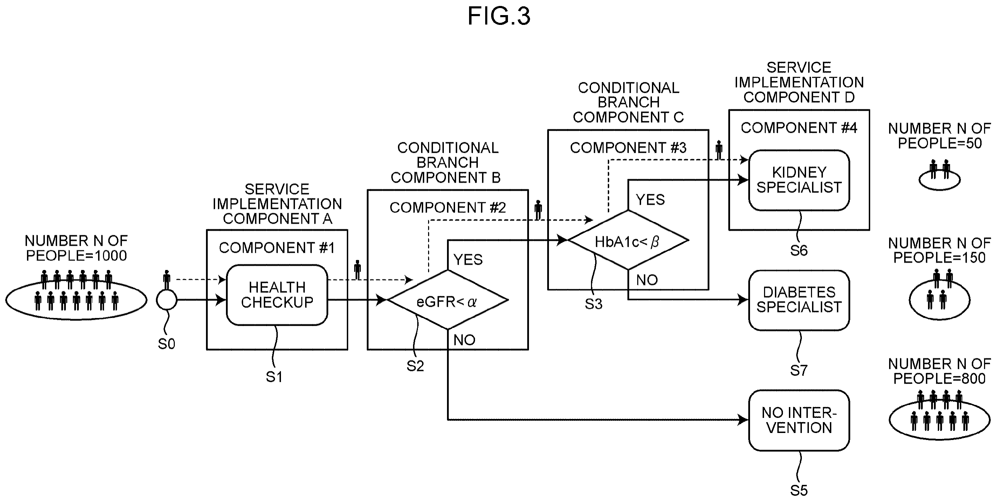

FIG. 3 is a diagram illustrating a specific example of a flow graph for a measure. As illustrated in FIG. 3, the measure is modeled as a workflow including a combination of conditional branch components, service implementation components, and the like. Then, the number of people who receive each service is output from a model trained by accumulating information and parameters on the way people flow from track record values when used for each conditional branch component. As a result, the local government can incorporate a measure suitable for the local government among measures implemented by other local governments in consideration of the resources of the local government.

In the example illustrated in FIG. 3, the number of people N=1000 is input in reference sign S0. In reference sign S1, component #1 as service implementation component A is set to "health checkup". In reference sign S2, component #2 as conditional branch component B is set to "eGFR<$\alpha$". When "eGFR<$\alpha$" is not satisfied (see the NO route in reference numeral S2), it is determined that "no intervention" is made by a specialist for relevant citizens as indicated in reference numeral S5.

On the other hand, when "eGFR<$\alpha$" is satisfied (see the YES route in reference numeral S2), component #3 as conditional branch component C is set to "HbA1c<$\beta$" as indicated in reference numeral S3. When "HbA1c<$\beta$" is satisfied (see the YES route in reference numeral S3), as indicated in reference numeral S6, component #4 as conditional branch component D is set to "kidney specialist", and it is determined that the intervention of "kidney specialist" is necessary for relevant citizens. On the other hand, when "HbA1c<$\beta$" is not satisfied (see the NO route in reference numeral S3), as indicated in reference numeral S7, it is determined that the intervention of "diabetes specialist" is necessary for relevant citizens.

In the example illustrated in FIG. 3, as indicated by arrows, the numbers of people flowing to components #1, #2, #3, and #4 in this order are predicted. For example, in the flow graph for the measure illustrated in FIG. 3, the result of assigning the number of people N=1000 to interventions Z2 to Z4 is as follows. 50 people are assigned to intervention Z2. 150 people are assigned to intervention Z3. Furthermore, 800 people are assigned to intervention Z4.

Here, another specific example of use of the flow graph for the measure will be described with reference to FIGS. 2 and 3. The server device 10 searches the flow graph for the measure using attribute information for a person in an organization to which the measure planner belongs. The server device 10 specifies a node into which the person is classified among nodes located at ends constituting the flow graph for the measure. As a result, the local government to which the measure has been applied can specify a medical institution to be recommended to the person in consideration of the health condition of the person belonging to the local government and the resources of the local government.

First, the server device 10 specifies a person included in an organization to which the measure planner belongs. For example, the server device 10 specifies a resident of a local government. Next, the server device 10 searches the flow graph for the measure output using the attribute information for the person in the organization to which the measure planner belongs, thereby specifying a node into which the specified person is classified among the nodes located at the ends constituting the flow graph for the measure. The attribute information is biological information specified by analyzing the body fluid of the person. The attribute information includes an estimated glomerular filtration rate, a hemoglobin A1c value, a urine protein value, and the like. The body fluid includes blood, lymph, tissue fluid (intertissue fluid, intercellular fluid, or interstitial fluid) sweat, tears, nasal mucus, urine, semen, vaginal fluid, amniotic fluid, milk, and the like.

At this time, the server device 10 specifies a node into which the person is classified by comparing the attribute information for the person with the condition included in the conditional branch component. The server device 10 specifies a node into which the specified person is classified among the nodes located at the ends. Then, the server device 10 sets the medical institution indicated by the specified classified node as a medical institution to be recommended to the specified person. The medical institution indicated by the node is a kidney specialist, a diabetes specialist, or the like.

Hereinafter, a measure that has already been implemented and has a track record may be referred to as an "existing measure", and a measure that is listed as an option to be implemented at the time of planning measures may be referred to as a "measure candidate". Furthermore, a flow graph for a measure may be abbreviated as a "measure flow". In addition, a measure flow corresponding to an existing measure among measure flows may be referred to as an "existing measure flow", and a measure flow corresponding to a measure candidate may be referred to as a "measure candidate flow".

<Data Infrastructure>

In the above-described data infrastructure, the measure flow may be shared in any framework. As just one example, the above-described data infrastructure can enable organizations around the world, for example, public organizations such as local governments, to share a measure flow.

Through the client terminal 30, the measure planner can refer to templates of existing measures from around the world shared by the above-described data infrastructure. For example, from the perspective of administrative (political) ease of implementation, a measure candidate can be generated by incorporating parts of flow graphs for a plurality of existing measures among templates collected in the data infrastructure. A flow graph for such a measure candidate may be automatically or manually generated through any technique.

<Automatic Generation of Measure Flow>

As just one example, a measure flow can be generated by a data-driven method such as optimal policy tree (OPT) or causal forest (CF). According to such a data-driven method, a flow graph for the most effective measure is preferentially generated, using training data in which services to which correct answers are assigned, for example, intervention labels, are allocated to feature amounts of objects used for determining branch conditions.

As another example, a measure flow can be generated by using a library from which a path structure serving as an element of a flow graph is extracted. The term "path" as used herein refers to a route formed by a series of branch nodes leading to a service on the flow graph. For example, the library of path structures is generated by listing an array of conditions defined for the respective branch nodes included in the path, that is, a branch sequence, from templates of existing measures collected in the above-described data infrastructure. A measure flow can be generated by searching for a path corresponding to a combination of target services from such a library of path structures.

<One Aspect of Problem>

As described in the background art section above, the evaluation of measures in the above-described conventional art tends to be biased to prediction of effects using Shapley additive explanation (SHAP) values or the like.

Since the evaluation of measures is biased to the effects of the measures in this manner, even if the most effective measure is selected, it is not always easy to change to the selected measure. For example, in order to implement the most effective measure, resources corresponding to that effect are required. However, the current resources may be insufficient to meet the required amount, it may be difficult to increase the resources, or the cost may be unacceptable even if the resources can be increased.

<One Aspect of Problem Solving Approach>

Therefore, in a case where a measure involves a person, attention is focused on the fact that the change affects not only the effect of the measure itself, but also the (consensus-based) judgement of the person who has determined the measure and the structure of implementation of the measure, as well as the range of the target of the resources, such as people and things that are targets of the measure.

Based on this perspective, in the present embodiment, a calculation function of calculating an evaluation value regarding a difference in structure of graph data, a difference in branch probability, a difference in effect value, or a difference in certainty factor of effect value is realized as an index when comparing flow graphs for two measures.

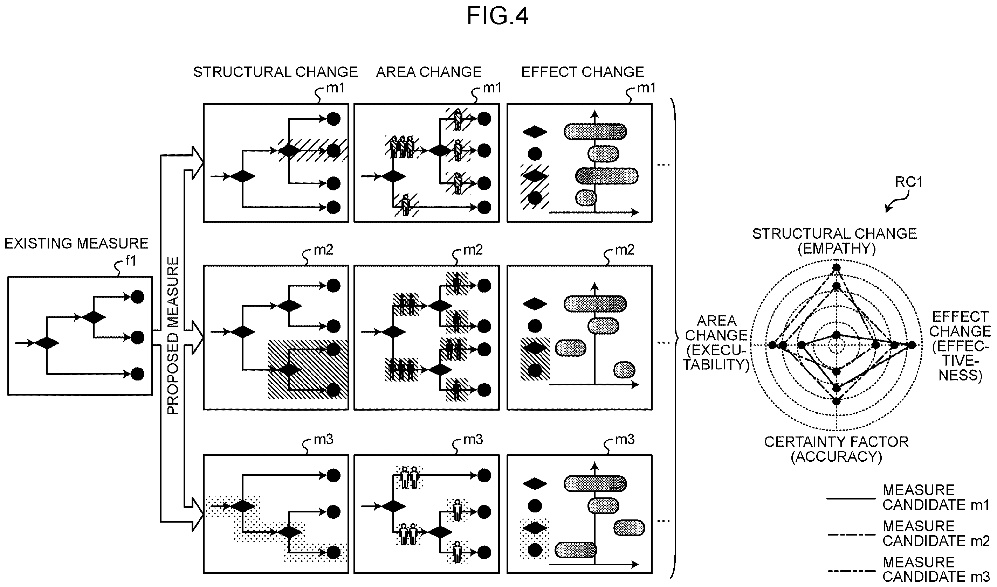

FIG. 4 is a diagram illustrating one aspect of the problem solving approach. FIG. 4 illustrates, as just one example, that evaluation values regarding structural change, area change, and effect change are calculated as indexes for comparing an existing measure flow f1 with three measure candidate flows m1 to m3.

As illustrated in FIG. 4, the structural change evaluation value can be calculated based on a cost when one of the two measure flows is converted into the other measure flow. For example, structural change evaluation values can be calculated by calculating tree edit distances, so-called TEDs, between the existing measure flow f1 and the three measure candidate flows m1 to m3.

The area change evaluation value can be calculated based on a difference in the number of objects allocated by the same branch node between two measure flows. For example, area change evaluation values can be calculated by calculating increases or decreases in the number of objects input to the same branch node between the existing measure flow f1 and the three measure candidate flows m1 to m3.

The effect change evaluation value can be calculated based on a difference in degree of influence of the condition at the branch node on the effect between the two measure flows. For example, effect change evaluation values can be calculated by calculating SHAP values of feature amounts defined as conditions at the branch nodes, that is, increases or decreases in degree of contribution to the effects, between the existing measure flow f1 and the three measure candidate flows m1 to m3.

These three types of evaluation values are merely example, and can be displayed as a chart in which evaluation values regarding each index are plotted on an axis corresponding to the index, for example, a radar chart RC1.

For example, in the radar chart RC1 illustrated in FIG. 4, in addition to the three indexes: structural change, area change, and effect change, the certainty factor in calculating the effect change evaluation value is plotted as a fourth index.

Such a radar chart RC1 enables multi-perspective evaluations between the existing measure flow f1 and the three measure candidate flows m1 to m3.

As one aspect, when effect change evaluation values are listed in descending order, an evaluation value between the existing measure flow f1 and the measure candidate flow m1, an evaluation value between the existing measure flow f1 and the measure candidate flow m2, and an evaluation value between the existing measure flow f1 and the measure candidate flow m3 are listed in this order. From such a result, it can be seen that, in terms of effectiveness, the change from the existing measure flow f1 to the measure candidate flow m1 is most satisfactory in the effect and efficiency of the entire measure.

As another aspect, when structural change evaluation values are listed in ascending order, an evaluation value between the existing measure flow f1 and the measure candidate flow m1, an evaluation value between the existing measure flow f1 and the measure candidate flow m2, and an evaluation value between the existing measure flow f1 and the measure candidate flow m3 are listed in this order. From such a result, it can be seen that, in terms of empathy, the change from the existing measure flow f1 to the measure candidate flow m1 is most psychologically acceptable.

As still another aspect, when area change evaluation values are listed in ascending order, an evaluation value between the existing measure flow f1 and the measure candidate flow m1, an evaluation value between the existing measure flow f1 and the measure candidate flow m2, and an evaluation value between the existing measure flow f1 and the measure candidate flow m3 are listed in this order. From such a result, it can be seen that, in terms of executability, the change from the existing measure flow f1 to the measure candidate flow m1 is easiest to execute.

These multi-perspective evaluations can prevent the measure candidate flow m1 from being immediately evaluated as the best because the effect change (effectiveness) of the change to the measure candidate flow m1 is evaluated as the best. That is, after confirming that the structural change (empathy) and the area change (executability) of the change to the measure candidate flow m1 are also evaluated as the best, it is possible to realize an evaluation that concludes that the measure candidate flow m1 is the best. In other words, when the structural change (empathy) and the area change (executability) of the change to the measure candidate flow m1 are evaluated as poor, it is possible to realize an evaluation that concludes that the measure candidate flow m1 is not the best.

Therefore, according to the calculation function according to the present embodiment, it is possible to evaluate an influence of a measure change. Although FIG. 4 illustrates an example in which evaluation values for the four indexes are calculated, it is not necessary to calculate evaluation values for all the four indexes, and for example, a structural change evaluation value or an area change evaluation value and an effect change evaluation value or a certainty factor evaluation value can be calculated. In addition, even in a case where only one of the structural change evaluation value or the area change evaluation value is calculated, it is possible to evaluate a measure without being biased to the effect of the measure, making it possible to evaluate an influence of a measure change.

<Configuration of Server Device 10>

FIG. 1 schematically illustrates blocks related to the data infrastructure included in the server device 10. As illustrated in FIG. 1, the server device 10 includes a communication control unit 11, a storage unit 13, and a control unit 15. Note that FIG. 1 merely illustrates excerpted functional units related to the above-described data infrastructure, and the server device 10 may include functional units other than those illustrated.

The communication control unit 11 is a functional unit that controls communication with other devices such as the client terminal 30. As just one example, the communication control unit 11 can be realized by a network interface card such as a LAN card. As one aspect, the communication control unit 11 receives a measure evaluation request for requesting execution of measure evaluation from the client terminal 30, or outputs a measure evaluation result to the client terminal 30.

The storage unit 13 is a functional unit that stores various types of data. As just one example, the storage unit 13 is realized by an internal, external, or auxiliary storage of the server device 10. For example, the storage unit 13 stores a measure database (DB) 13A, a feature amount DB 13B, and an effect DB 13C.

The measure DB 13A is a database that stores a set of measure flows. The feature amount DB 13B is a database that stores feature amounts related to objects that are targets of measures. For example, in an example of a measure in the medical field, from the aspect in which residents are allocated to services, test values for each test item included in health checkup results are stored as feature amounts used for determining conditions at branch nodes in the feature amount DB 13B. Such feature amounts of residents may be stored in an organizational unit, for example, in a local government unit, to which a measure planner having an account for using the above-described data infrastructure belongs. The effect DB 13C is a database that stores effects when objects are allocated to the services of the measure flows. For example, for each service, that is, for each intervention, the effect of the intervention, for example, the degree of improvement in kidney function, is stored in the effect DB 13C in an example of a measure flow regarding medical care follow-up for chronic kidney disease (CKD).

The control unit 15 is a functional unit that performs the overall control of the server device 10. For example, the control unit 15 can be realized by a hardware processor. Alternatively, the control unit 15 may be realized by hard-wired logic. As illustrated in FIG. 1, the control unit 15 includes a reception unit 15A, an acquisition unit 15B, a calculation unit 15C, and an output unit 15D.

The reception unit 15A is a processing unit that receives various requests from the client terminal 30. As just one example, the reception unit 15A can receive a measure evaluation request for requesting execution of measure evaluation from the client terminal 30. As just one example, such a measure evaluation request may include designation of a pair of measure flows to be compared. For example, it is possible to designate an existing measure flow that has already been implemented and has a track record and M (any natural number) measure candidate flows from among the measure flows stored in the measure DB 13A. Furthermore, the measure evaluation request may include designation of an object that is a target of the measure or an organization to which the object belongs, for example, a local government.

The acquisition unit 15B is a processing unit that acquires a measure flow from the measure DB 13A. As just one example, the acquisition unit 15B acquires the measure flows designated in the measure evaluation request received by the reception unit 15A, that is, the existing measure flow and the M measure candidate flows, from the measure DB 13A. Furthermore, the acquisition unit 15B can acquire the feature amount of the object designated in the measure evaluation request from the feature amount DB 13B. In addition, the acquisition unit 15B can acquire, from the effect DB 13C, effects when the feature amount of the object designated in the measure evaluation request is applied to the measure flows designated in the measure evaluation request.

The calculation unit 15C is a processing unit that calculates an evaluation value regarding each index. As just one example, the calculation unit 15C calculates evaluation values regarding N (any natural number) indexes between the existing measure flow and each of the M measure candidate flows.

As one of such indexes, the calculation unit 15C can calculate an evaluation value regarding structural change between the existing measure flow and the measure candidate flow. For example, the calculation unit 15C calculates the evaluation value regarding structural change based on the cost when one of the two measure flows is converted into the other measure flow. As just one example, the calculation unit 15C can calculate the structural change evaluation value by calculating a tree edit distance $\Delta_{str}(T_1, T_2)$ between an existing measure flow $T_1$ and a measure candidate flow $T_2$ according to the following Formula (1).

$$\Delta_{str}(T_1, T_2) = \sum_{(i,j) \in M} cost(T_1 \to T_2)$$
$$+ \sum_{i \in M_{del}} cost(T_1 \to \Lambda) + \sum_{i \in M_{ins}} cost(\Lambda \to T_2) \quad \cdots (1)$$

Here, "cost $(T_1 \to T_2)$" in the above Formula (1) indicates a cost when the node of the existing measure flow $T_1$ is replaced with the node of the measure candidate flow $T_2$. Furthermore, "cost $(T_1 \to \Lambda)$" in the above Formula (1) indicates a cost when the node of the existing measure flow $T_1$ is deleted. Furthermore, "cost $(\Lambda \to T_2)$" in the above Formula (1) indicates a cost when the node of the measure candidate flow $T_2$ is added.

As another index, the calculation unit 15C can calculate an evaluation value regarding area change between the existing measure flow and the measure candidate flow. For example, the calculation unit 15C can calculate the evaluation value regarding area change based on the difference in the number of objects allocated by the same branch node between the two measure flows. As just one example, the calculation unit 15C can calculate the area change evaluation value by calculating increases or decreases $\Delta_{cov}(T_1, T_2)$ in the number of objects input to the same branch node between the existing measure flow $T_1$ and the measure candidate flow $T_2$ according to the following Formula (2).

$$\Delta_{cov}(T_1, T_2) = \sum_{k \in (i,j)} \left( N_{2,k}(D) - N_{1,k}(D) \right) \quad \cdots (2)$$

Here, "$N_{2,k}(D)$" in the above Formula (2) refers to the number of people input to a branch node k when a data set D including feature amounts of object groups belonging to any cluster, for example, test values for each test item of resident groups belonging to a local government, is applied to the measure candidate flow $T_2$. Furthermore, "$N_{1,k}(D)$" in the above Formula (2) refers to the number of people input to the branch node k when the above-described data set D is applied to the existing measure flow $T_1$.

As another index, the calculation unit 15C can calculate an evaluation value regarding effect change between the existing measure flow and the measure candidate flow. For example, the calculation unit 15C can calculate the evaluation value regarding effect change based on a difference in degree of influence of the condition at the branch node on the effect between the two measure flows. As just one example, the calculation unit 15C can calculate the effect change evaluation value by calculating an SHAP value of the feature amount defined as a condition at the branch node between the existing measure flow and the measure candidate flow, that is, an increase/decrease $\Delta_{out}(T_1, T_2)$ in degree of contribution to the effect, according to the following Formula (3).

$$\Delta_{out}(T_1, T_2) = \frac{1}{J} \sum_{j \in T_2} \phi_j(D) - \frac{1}{I} \sum_{i \in T_1} \phi_i(D) \qquad \cdots (3)$$

Here, "$\phi_j(D)$" in the above Formula (3) refers to a degree of contribution to the effect at a branch node j when a data set D including feature amounts of object groups belonging to any cluster, for example, test values for each test item of resident groups belonging to a local government, is applied to the measure candidate flow $T_2$. In addition, "$\phi_i(D)$" in the above Formula (3) indicates a degree of contribution to the effect at the branch node i when the above-described data set D is applied to the existing measure flow $T_1$.

As another index, the calculation unit 15C can calculate an evaluation value for an index regarding reliability, such as error, variation, accuracy, loss, or convergence rate, of an existing measure generation model. As just one example, as an example of the index of the measure generation model, it is possible to calculate a difference in mean regret between the existing measure flow and the measure candidate flow. For example, the mean regret of the existing measure flow can be calculated according to the following Formula (4). "$\max(Y_t(l))$" in the following Formula (4) refers to an effect in a case where the most effective measure flow is selected when a possible measure flow T is applied to a data set l including feature amounts of object groups belonging to any cluster, and ($Y_{t1}(l)$) refers to an effect when the existing measure flow $T_1$ is applied. In addition, since the mean regret of the measure candidate flow can also be calculated in the same manner as in the following Formula (4), a difference between the mean regret of the existing measure flow and the mean regret of the measure candidate flow can also be calculated.

$$\Delta_{mr}(T_1) = \frac{1}{L} \left[ \sum_{l \in D} \max_{t \in T}(Y_t(l)) - \sum_{l \in D} Y_{T_1}(l) \right] \qquad \cdots (4)$$

In addition, the calculation unit 15C can calculate an evaluation value for an index in a digitized measure. For example, the index in the digitized measure refers to an index used when quantitatively comparing formalized rules, documents, and the like. For example, an evaluation index used in Rules as Code, legal document comparison, and the like may be used as the index in the digitized measure.

The output unit 15D is a processing unit that outputs various types of information to the client terminal 30. As just one example, the output unit 15D outputs the evaluation values for the N indexes calculated for each of the M measure candidate flows by the calculation unit 15C to the client terminal 30.

<Specific Example (1) in Which Evaluation Values Are Output>

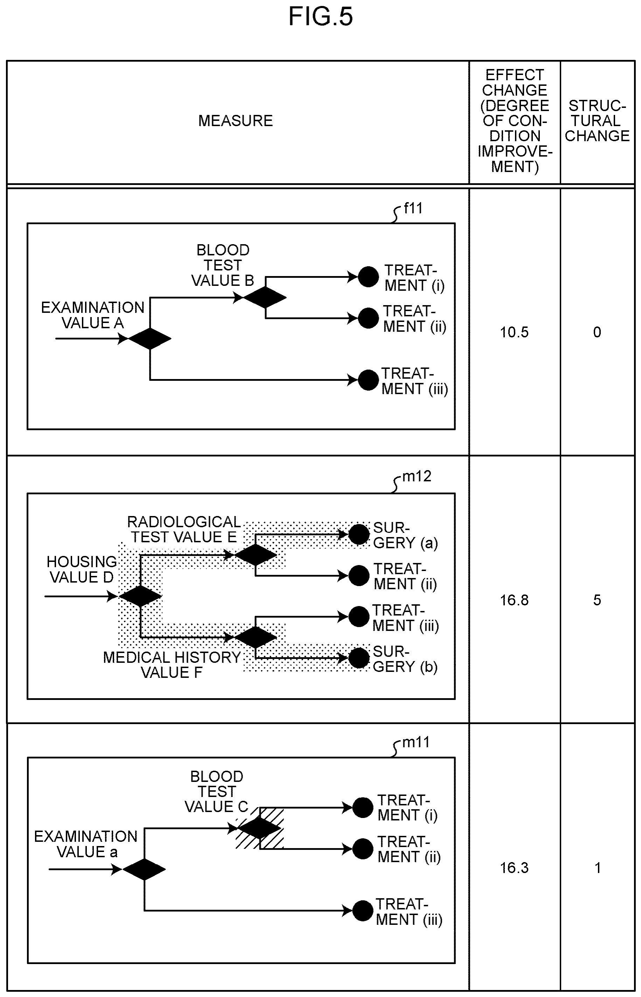

FIG. 5 is a diagram (1) illustrating an example in which evaluation values are displayed. FIG. 5 illustrates an example in which evaluation values for two indexes: effect changes and structural changes between an existing measure flow f11 and two measure candidate flows m11 and m12 are displayed on the client terminal 30. As illustrated in FIG. 5, the evaluation value for effect change between the existing measure flow f11 and the measure candidate flow m12 is displayed as "16.8". On the other hand, the evaluation value for effect change between the existing measure flow f11 and the measure candidate flow m11 is displayed as "16.3".

Therefore, if the effect change evaluation values are only considered, there is a high possibility that the measure candidate flow m12 is selected as a measure flow to be changed from the existing measure flow f11, because the effect is greatest when the existing measure flow f11 is changed to the measure candidate flow m12.

Even if the measure candidate flow m12 is selected in this manner, it may be difficult to change the existing measure flow f11 to the measure candidate flow m12. This is because the evaluation value for structural change between the existing measure flow f11 and the measure candidate flow m12 is as large as "5". For example, when the existing measure flow f11 is changed to the measure candidate flow m12, permission and adjustment between parties concerned are required to change the reference data and the medical practice, and thus, the burden of changing to the measure candidate flow m12 is large.

However, according to the example illustrated in FIG. 5, not only the effect change evaluation values but also the structural change evaluation values are displayed on the client terminal 30. Therefore, it can also be confirmed that the evaluation value for structural change between the existing measure flow f11 and the measure candidate flow m12 is "5", while the evaluation value for structural change between the existing measure flow f11 and the measure candidate flow m11 is "1".

Therefore, the measure planner can confirm that there is no large difference in effect change evaluation value, while there is a large difference in structural change evaluation value, between the change from the existing measure flow f11 to the measure candidate flow m12 and the change from the existing measure flow f11 to the measure candidate flow m11.

For example, when the existing measure flow f11 is changed to the measure candidate flow m11, the structural change evaluation value is as small as "1", and thus, it can be seen that the determination and execution steps do not need to be greatly changed from the existing measure. Furthermore, it can also be seen that, when the existing measure flow f11 is changed to the measure candidate flow m11, an effect equivalent to that when the existing measure flow f11 is changed to the measure candidate flow m12 can be obtained.

By making such multi-perspective evaluations possible, it is possible to easily select the measure candidate flow m11, which is a globally optimal solution, without falling into a situation where the measure candidate flow m12, which is a locally optimal solution, is selected, as a measure flow to be changed from the existing measure flow f11.

<Specific Example (2) in Which Evaluation Values Are Output>

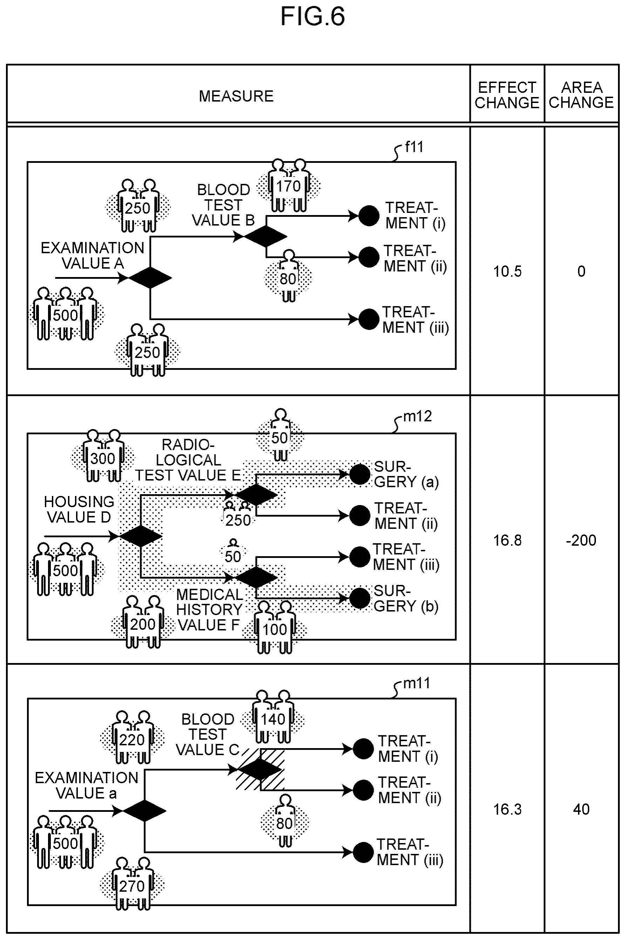

FIG. 6 is a diagram (2) illustrating an example in which evaluation values are displayed. FIG. 6 illustrates an example in which evaluation values for two indexes: effect changes and area changes between an existing measure flow f11 and two measure candidate flows m11 and m12 are displayed on the client terminal 30.

As illustrated in FIG. 6, the evaluation value for effect change between the existing measure flow f11 and the measure candidate flow m12 is displayed as "16.8", and the evaluation value for effect change between the existing measure flow f11 and the measure candidate flow m11 is displayed as "16.3". Further, the evaluation value for area change between the existing measure flow f11 and the measure candidate flow m12 is displayed as "-200", and the evaluation value for area change between the existing measure flow f11 and the measure candidate flow m11 is displayed as "40".

In this manner, not only the effect change evaluation values but also the area change evaluation values are displayed on the client terminal 30. Therefore, the measure planner can confirm that there is no large difference in effect change evaluation value, while there is a large difference in area change evaluation value, between the change from the existing measure flow f11 to the measure candidate flow m12 and the change from the existing measure flow f11 to the measure candidate flow m11.

For example, when the existing measure flow f11 is changed to the measure candidate flow m12, the area change evaluation value is as large as "-200", and thus, it can be seen that it is necessary to greatly change the amount of change in the number of people who are targets of tests or treatments or the number of people who are to be subjected to tests or treatments from the existing measure. On the other hand, when the existing measure flow f11 is changed to the measure candidate flow m11, the area change evaluation value is as small as "40", and thus, it can be seen that it is not necessary to greatly change the amount of change in the number of people who are targets of tests or treatments or the number of people who are to be subjected to tests or treatments from the existing measure. Furthermore, when the existing measure flow f11 is changed to the measure candidate flow m11, an effect equivalent to that when the existing measure flow f11 is changed to the measure candidate flow m12 can be obtained.

By making such multi-perspective evaluations possible, it is possible to easily select the measure candidate flow m11, which is a globally optimal solution, without falling into a situation where the measure candidate flow m12, which is a locally optimal solution, is selected, as a measure flow to be changed from the existing measure flow f11.

<Example of Numerical Calculation of Area Change Evaluation Values>

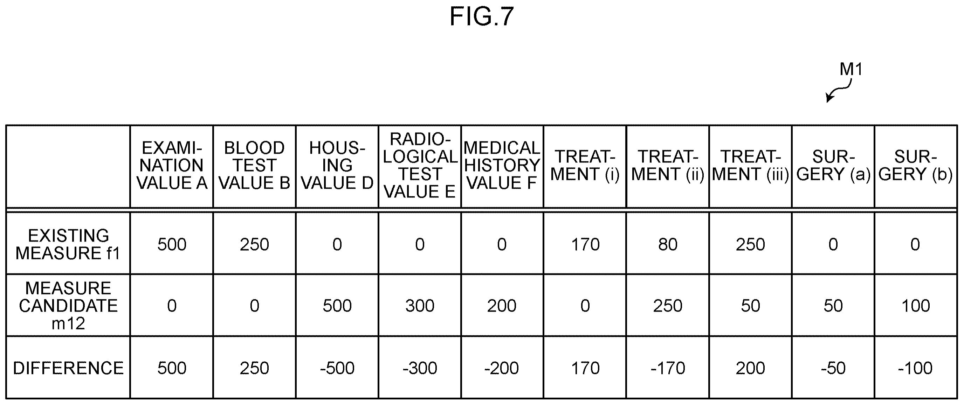

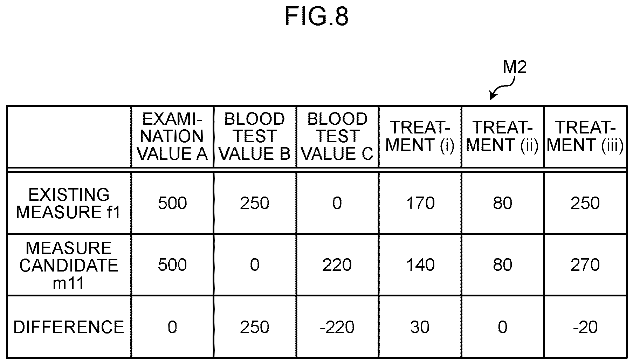

Here, examples of numerical calculations of the area change evaluation values illustrated in FIG. 6 will be described. FIGS. 7 and 8 are schematic diagrams (1) for explaining an example of a numerical calculation of an area change evaluation value. FIG. 7 illustrates a map M1 indicating the numbers of people input to the branch nodes in the existing measure flow f11 and the measure candidate flow m12 for all the types of feature amounts defined in the conditions at the branch nodes of the existing measure flow f11 and the measure candidate flow m12. On the other hand, FIG. 8 illustrates a map M2 indicating the numbers of people input to the branch nodes in the existing measure flow f11 and the measure candidate flow m11 for all the types of feature amounts defined in the conditions at the branch nodes of the existing measure flow f11 and the measure candidate flow m11. Note that, in the map M1 illustrated in FIG. 7, for a type of feature amount that exists at a branch node of one measure flow and does not exist at a branch node of the other measure flow, the feature amount of the measure flow where the feature amount does not exist is set to zero.

As illustrated in FIG. 7, an OR set of types of feature amounts defined in the conditions at the branch nodes between the existing measure flow f11 and the measure candidate flow m12 includes examination value A, blood test value B, housing value B, radiological test value E, medical history value F, treatment (i), treatment (ii), treatment (iii), surgery (a), and surgery (b). For each of these types of feature amounts, a difference is calculated by subtracting the number of people input to the branch node in the measure candidate flow m12 from the number of people input to the branch node in the existing measure flow f11. In this manner, the sum "500+250-500-300-200+170-170+200-50-100" of the differences calculated for all the types of feature amounts is calculated. As a result, the evaluation value for area change between the existing measure flow f11 and the measure candidate flow m12 is calculated as "-200".

FIG. 8 is a schematic diagram (2) for explaining an example of a numerical calculation of an area change evaluation value. FIG. 8 illustrates a map M2 indicating the numbers of people input to the branch nodes in the existing measure flow f11 and the measure candidate flow m11 for all the types of feature amounts defined in the conditions at the branch nodes of the existing measure flow f11 and the measure candidate flow m11. Note that, in the map M2 illustrated in FIG. 8, for a type of feature amount that exists at a branch node of one measure flow and does not exist at a branch node of the other measure flow, the feature amount of the measure flow where the feature amount does not exist is set to zero.

As illustrated in FIG. 8, an OR set of types of feature amounts defined in the conditions at the branch nodes between the existing measure flow f11 and the measure candidate flow m11 includes examination value A, blood test value B, blood test value C, treatment (i), treatment (ii), and treatment (iii). For each of these types of feature amounts, a difference is calculated by subtracting the number of people input to the branch node in the measure candidate flow m11 from the number of people input to the branch node in the existing measure flow f11. In this manner, the sum "0+250-220+30+0-20" of the differences calculated for all the types of feature amounts is calculated. As a result, the evaluation value for area change between the existing measure flow f11 and the measure candidate flow m12 is calculated as "40".

<Specific Example (3) in Which Evaluation Values Are Output>

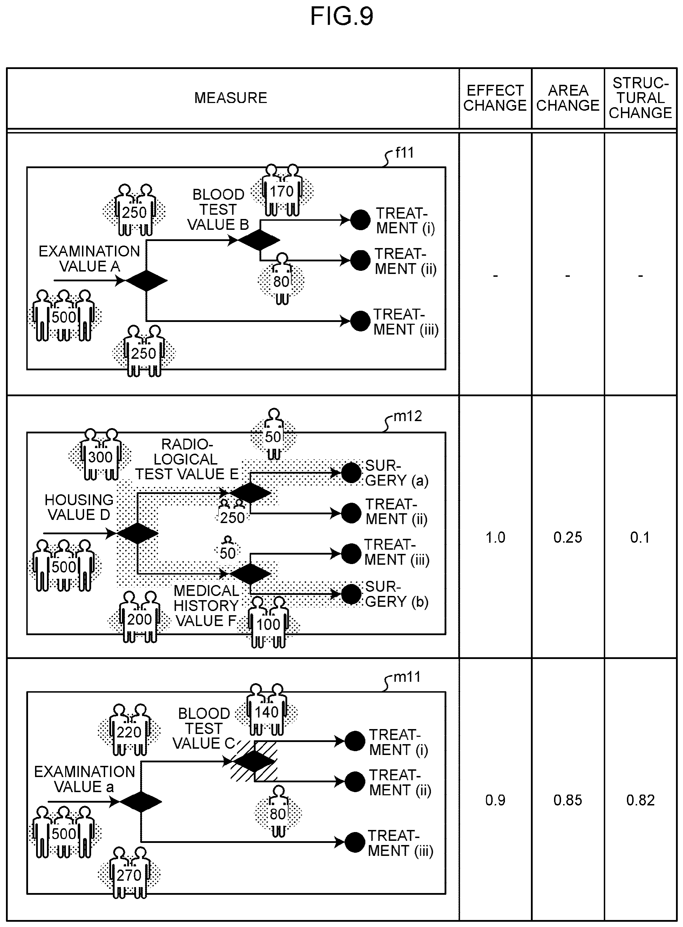

FIG. 9 is a diagram (3) illustrating an example in which evaluation values are displayed. FIG. 9 illustrates an example in which evaluation values for three indexes: effect changes, area changes, and structural changes between an existing measure flow f11 and two measure candidate flows m11 and m12 are displayed on the client terminal 30.

As illustrated in FIG. 9, the evaluation values for the three indexes: effect change, area change, and structural change are normalized to a numerical range from 0 to 1. Such normalization enables comparison between the indexes with a unified scale of evaluation values.

Further, the area change evaluation values and the structural change evaluation values are normalized to values of which the magnitudes have a positive correlation with the levels of the evaluations. That is, the smaller the change in area (number of people) between the existing measure flow and the measure candidate flow, the larger the area change evaluation value and the higher the evaluation. In addition, the smaller the structural change between the existing measure flow and the measure candidate flow, the larger the structural change evaluation value and the higher the evaluation. Such normalization eliminates the need for back calculations to unify an index having a positive correlation between a magnitude of an evaluation value and a level of an evaluation and an index having a negative correlation between a magnitude of an evaluation value and a level of an evaluation into one of the positive correlation and the negative correlation, making it possible to more quickly evaluate an influence of a measure change.

<Specific Example (4) in Which Evaluation Values Are Output>

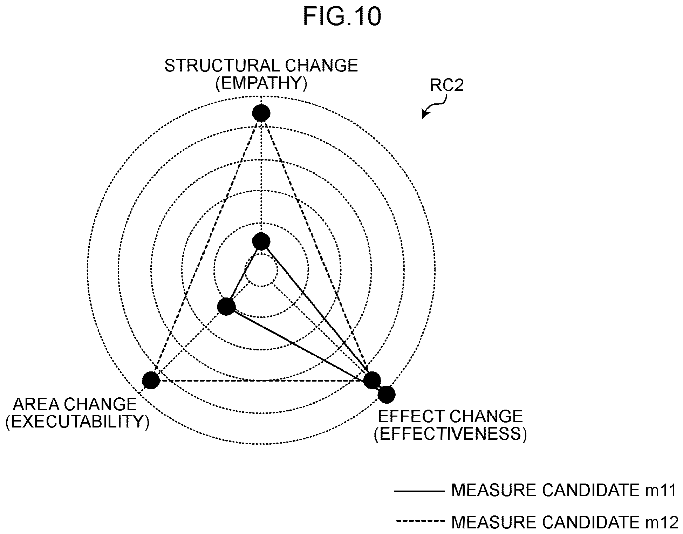

FIG. 10 is a diagram (4) illustrating an example in which evaluation values are displayed. FIG. 10 illustrates an example in which a radar chart RC2, in which the three types of evaluation values for effect change, area change, and structural change illustrated in FIG. 9 are plotted on the axes corresponding to the respective indexes, is displayed on the client terminal 30. As illustrated in FIG. 10, an overall evaluation of all the multi-perspective indexes can be presented based on the size of the area of the polygon, thereby making it possible to intuitively grasp superiority and inferiority between the measure candidate flows.

<Flow of Processing>

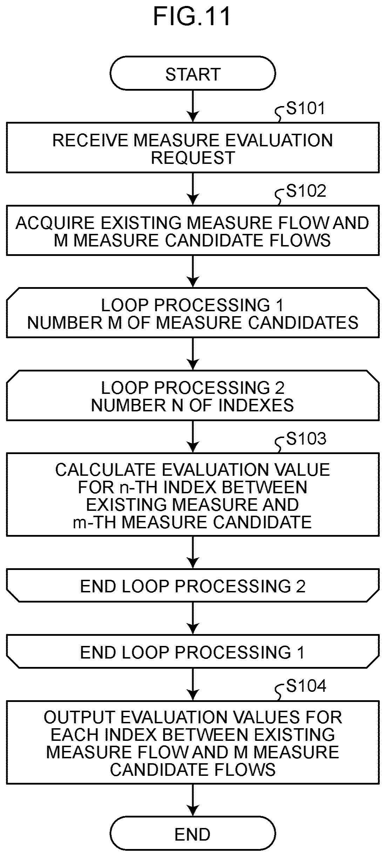

FIG. 11 is a flowchart illustrating calculation processing steps. This processing can be executed as just one example when a measure evaluation request for requesting execution of measure evaluation is received from the client terminal 30.

As illustrated in FIG. 11, when the reception unit 15A receives a measure evaluation request (step S101), the acquisition unit 15B executes the following processing. That is, the acquisition unit 15B acquires measure flows designated in the measure evaluation request received in step S101, that is, an existing measure flow and M measure candidate flows, from the measure DB 13A (step S102).

Subsequently, the calculation unit 15C executes loop processing 1 of repeating processing of step S103 as many as the number of times corresponding to the number M of measure candidate flows. Furthermore, the calculation unit 15C executes loop processing 2 of repeating processing of step S103 as many as the number of times corresponding to the number N of types of indexes between the existing measure flow and the m-th measure candidate flow.

That is, the calculation unit 15C calculates an evaluation value regarding the n-th index between the existing measure flow and the m-th measure candidate flow (step S103).

By repeating such loop processing 2, evaluation values are calculated for each of the N indexes. Furthermore, by repeating the loop processing 1, evaluation values for the N indexes are calculated for each of the M measure candidate flows.

Thereafter, the output unit 15D outputs the evaluation values for the N indexes calculated for each of the M measure candidate flows in step S103 to the client terminal 30 (step S104), and ends the processing.

<One Aspect of Effect>

As described above, the server device 10 according to the present embodiment calculates an evaluation value regarding a difference in structure of graph data, a difference in branch probability, a difference in effect value, or a difference in certainty factor of effect value as an index when comparing two flow graphs for an existing measure and a measure candidate. By outputting the evaluation value for each of the plurality of indexes among the evaluation values for the indexes, multi-perspective evaluations can be performed between the flow graphs for the existing measure and the measure candidate. Therefore, the server device 10 according to the present embodiment can evaluate an influence of the measure change.

<Second Embodiment>

Although the embodiment relating to the disclosed device has been described so far, the present invention may be embodied in various different forms other than the above-described embodiment. Therefore, other embodiments that fall within the present invention will be described below.

<Calculation of Overall Evaluation Value>

In the first embodiment described above, it is exemplified that an evaluation value for each of the plurality of indexes, such as effect change, area change, and structural change, is output, but the present invention is not limited thereto. For example, the calculation unit 15C can calculate one overall evaluation value from the evaluation values for the plurality of indexes by executing statistical processing, such as arithmetic averaging or weighted averaging, on the evaluation values for the plurality of indexes.

<Narrowing Down of Measure Candidates To Be Output>

Furthermore, in the first embodiment described above, it has been exemplified that all of the measure candidate flows designated in the measure evaluation request are targets to be output, but the measure candidate flows can be narrowed down to measure candidate flows in which the evaluation values for one or more indexes satisfy specific conditions, and some of the measure candidate flows can be output. For example, the output unit 15D can output a measure candidate flow in which the evaluation value for the specific index is equal to or more than the threshold value or equal to or less than the threshold value, or a measure candidate flow in which the evaluation value for each of the plurality of indexes is equal to or more than the threshold value or equal to or less than the threshold value. In addition, the output unit 15D can output measure candidate flows in which the evaluation values for the specific index fall within the top specific number, for example, within the top three, or measure candidate flows in which the evaluation values of each of the plurality of indexes falls within the top specific number, for example, within the top three.

<Distribution and Integration>

In addition, it is not required that each component of each device illustrated in the drawings be physically configured as illustrated in the drawings. That is, the specific distributed and integrated form of each device is not limited to the illustrated form, and all or part of each device can be functionally or physically distributed and integrated in any unit depending on various loads, usage conditions, and the like. For example, the reception unit 15A, the acquisition unit 15B, the calculation unit 15C, or the output unit 15D may be connected via a network as an external device of the server device 10. In addition, the reception unit 15A, the acquisition unit 15B, the calculation unit 15C, or the output unit 15D may be provided in a separate device connected via a network for cooperation to implement the function of the server device 10.

<Hardware Configuration>

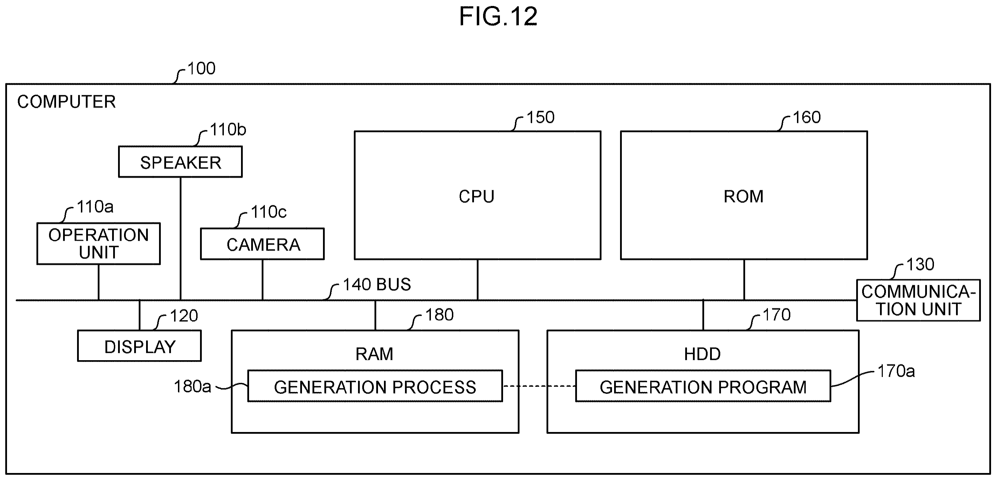

In addition, the various types of processing described in the above embodiments can be realized by executing a program prepared in advance on a computer such as a personal computer or a workstation. Therefore, an example of a computer that executes a calculation program having the same functions as those in the first and second embodiments will be described below with reference to FIG. 12.

FIG. 12 is a diagram illustrating an example of a hardware configuration. As illustrated in FIG. 12, a computer 100 includes an operation unit 110a, a speaker 110b, a camera 110c, a display 120, and a communication unit 130. The computer 100 further includes a CPU 150, a ROM 160, an HDD 170, and a RAM 180. These units 110 to 180 are connected to each other via a bus 140.

As illustrated in FIG. 12, the HDD 170 stores a calculation program 170a that exerts functions similar to those of the reception unit 15A, the acquisition unit 15B, the calculation unit 15C, and the output unit 15D described in the first embodiment described above. The calculation program 170a may be integrated or separated similarly to the components of the reception unit 15A, the acquisition unit 15B, the calculation unit 15C, and the output unit 15D illustrated in FIG. 1. That is, it is not necessary that the HDD 170 store all the data described in the first embodiment described above, as long as data to be used for processing may be stored in the HDD 170.

Under such an environment, the CPU 150 reads the calculation program 170a from the HDD 170, and then loads the calculation program 170a into the RAM 180. As a result, as illustrated in FIG. 12, the calculation program 170a functions as a calculation process 180a. The calculation process 180a loads various types of data read from the HDD 170 into an area assigned to the calculation process 180a among storage areas included in the RAM 180, and executes various types of processing using the loaded various types of data. For example, the processing illustrated in FIG. 11 is included as an example of the processing executed by the calculation process 180a. Note that, in the CPU 150, it is not necessary that all the processing units described above in the first embodiment operate, as long as a processing unit corresponding to processing to be executed is virtually realized.

Note that it is not necessary that the calculation program 170a be stored in the HDD 170 or the ROM 160 from the beginning. For example, each program is stored in a "portable physical medium" such as a flexible disk inserted into the computer 100, a so-called FD, CD-ROM, DVD disk, magneto-optical disk, or IC card. Then, the computer 100 may acquire and execute each program from the portable physical medium. In addition, each program may be stored in another computer, a server device, or the like connected to the computer 100 via a public line, the Internet, a LAN, a WAN, or the like, and the computer 100 may acquire and execute each program therefrom.

Reference Signs List

10      SERVER DEVICE

11      COMMUNICATION CONTROL UNIT

13      STORAGE UNIT

13A     MEASURE DB

13B     FEATURE AMOUNT DB

13C     EFFECT DB

15      CONTROL UNIT

15A     RECEPTION UNIT

15B     ACQUISITION UNIT

15C     CALCULATION UNIT

15D     OUTPUT UNIT

30      CLIENT TERMINAL

**Claims**

1.  A generation method executed by a computer, the generation method comprising:

        acquiring a first measure and a second measure configured from graph data including a plurality of conditional branches coupled by a directed edge and nodes coupled to destinations to which each of the plurality of conditional branches is branched;

generating information regarding a first difference between the acquired first and second measures, the first difference being at least one of a difference in structure of graph data and a difference in branch probability of an object assigned to a node of the conditional branch; and

generating correspondence information in which the generated information regarding the first difference is associated with information regarding a second difference between the acquired first and second measures, the second difference being at least one of a difference in effect value and a difference in certainty factor.

2. The generation method according to claim 1, wherein processing of calculating the first difference includes processing of calculating the difference in structure of graph data based on a cost when the measure in one of the graph data for the first measure and the graph data for the second measure is converted into the measure in the other one.

3. The generation method according to claim 2, wherein the cost corresponds to a tree edit distance.

4. The generation method according to claim 1, wherein processing of calculating the first difference includes processing of calculating the difference in branch probability based on a difference in the number of objects allocated by a node of the same conditional branch between the first measure and the second measure.

5. The generation method according to claim 1, wherein processing of calculating the second difference includes processing of calculating the difference in effect value based on a difference in degree of influence of the plurality of conditional branches on the effect value between the first measure and the second measure.

6. The generation method according to claim 1, wherein the computer further executes outputting a radar chart in which at least one of the difference in structure of graph data, the difference in branch probability, the difference in effect value, and the difference in certainty factor is plotted in a polygonal shape.

7. A generation program causing a computer to execute:

acquiring a first measure and a second measure configured from graph data including a plurality of conditional branches coupled by a directed edge and nodes coupled to destinations to which each of the plurality of conditional branches is branched;

generating information regarding a first difference between the acquired first and second measures, the first difference being at least one of a difference in structure of graph data and a difference in branch probability of an object assigned to a node of the conditional branch; and

generating correspondence information in which the generated information regarding the first difference is associated with information regarding a second difference between the acquired first and second measures, the second difference being at least one of a difference in effect value and a difference in certainty factor.

8. The generation program according to claim 7, wherein processing of calculating the first difference includes processing of calculating the difference in structure of graph data based on a cost when the measure in one of the graph data for the first measure and the graph data for the second measure is converted into the measure in the other one.

9. The generation program according to claim 8, wherein the cost corresponds to a tree edit distance.

10. The generation program according to claim 7, wherein processing of calculating the first difference includes processing of calculating the difference in branch probability based on a difference in the number of objects allocated by a node of the same conditional branch between the first measure and the second measure.

11. The generation program according to claim 7, wherein processing of calculating the second difference includes processing of calculating the difference in effect value based on a difference in degree of influence of the plurality of conditional branches on the effect value between the first measure and the second measure.

12. The generation program according to claim 7, wherein the computer further executes outputting a radar chart in which at least one of the difference in structure of graph data, the difference in branch probability, the difference in effect value, and the difference in certainty factor is plotted in a polygonal shape.

13. An information processing apparatus comprising a control unit that executes:

acquiring a first measure and a second measure configured from graph data including a plurality of conditional branches coupled by a directed edge and nodes coupled to destinations to which each of the plurality of conditional branches is branched;
generating information regarding a first difference between the acquired first and second measures, the first difference being at least one of a difference in structure of graph data and a difference in branch probability of an object assigned to a node of the conditional branch; and
generating correspondence information in which the generated information regarding the first difference is associated with information regarding a second difference between the acquired first and second measures, the second difference being at least one of a difference in effect value and a difference in certainty factor.

14. The information processing apparatus according to claim 13, wherein processing of calculating the first difference includes processing of calculating the difference in structure of graph data based on a cost when the measure in one of the graph data for the first measure and the graph data for the second measure is converted into the measure in the other one.

15. The information processing apparatus according to claim 14, wherein the cost corresponds to a tree edit distance.

16. The information processing apparatus according to claim 13, wherein processing of calculating the first difference includes processing of calculating the difference in branch probability based on a difference in the number of objects allocated by a node of the same conditional branch between the first measure and the second measure.

17. The information processing apparatus according to claim 13, wherein processing of calculating the second difference includes processing of calculating the difference in effect value based on a difference in degree of influence of the plurality of conditional branches on the effect value between the first measure and the second measure.

18. The information processing apparatus according to claim 13, wherein the control unit further executes outputting a radar chart in which at least one of the difference in structure of graph data, the difference in branch probability, the difference in effect value, and the difference in certainty factor is plotted in a polygonal shape.

FIG.1

SERVER DEVICE 10

STORAGE UNIT 13

MEASURE DB 13A

FEATUREAMOUNT DB 13B

EFFECT DB 13C

CONTROL UNIT 15

RECEPTION UNIT 15A

ACQUISITION UNIT 15B

CALCULATION UNIT 15C

OUTPUT UNIT 15D

COMMUNICATION CONTROL UNIT 11

NW

CLIENT TERMINAL 30

# FIG.2

FIG.3

FIG.4

# FIG.5

| MEASURE | EFFECT CHANGE (DEGREE OF CONDITION IMPROVEMENT) | STRUCTURAL CHANGE |
|---|---|---|
| f11 — EXAMINATION VALUE A → BLOOD TEST VALUE B → TREATMENT (i) / TREATMENT (ii) / TREATMENT (iii) | 10.5 | 0 |
| m12 — HOUSING VALUE D → RADIOLOGICAL TEST VALUE E / MEDICAL HISTORY VALUE F → SURGERY (a) / TREATMENT (ii) / TREATMENT (iii) / SURGERY (b) | 16.8 | 5 |
| m11 — EXAMINATION VALUE a → BLOOD TEST VALUE C → TREATMENT (i) / TREATMENT (ii) / TREATMENT (iii) | 16.3 | 1 |

# FIG.6

| MEASURE | EFFECT CHANGE | AREA CHANGE |
|---|---|---|
| | 10.5 | 0 |
| | 16.8 | -200 |
| | 16.3 | 40 |

# FIG.7

M1

| | EXAMI-NATION VALUE A | BLOOD TEST VALUE B | HOUS-ING VALUE D | RADIO-LOGICAL TEST VALUE E | MEDICAL HISTORY VALUE F | TREAT-MENT (i) | TREAT-MENT (ii) | TREAT-MENT (iii) | SUR-GERY (a) | SUR-GERY (b) |
|---|---|---|---|---|---|---|---|---|---|---|
| EXISTING MEASURE f1 | 500 | 250 | 0 | 0 | 0 | 170 | 80 | 250 | 0 | 0 |
| MEASURE CANDIDATE m12 | 0 | 0 | 500 | 300 | 200 | 0 | 250 | 50 | 50 | 100 |
| DIFFERENCE | 500 | 250 | -500 | -300 | -200 | 170 | -170 | 200 | -50 | -100 |

EP 4 730 234 A1

# FIG.8

M2

|  | EXAMI-NATION VALUE A | BLOOD TEST VALUE B | BLOOD TEST VALUE C | TREAT-MENT (i) | TREAT-MENT (ii) | TREAT-MENT (iii) |
|---|---|---|---|---|---|---|
| EXISTING MEASURE f1 | 500 | 250 | 0 | 170 | 80 | 250 |
| MEASURE CANDIDATE m11 | 500 | 0 | 220 | 140 | 80 | 270 |
| DIFFERENCE | 0 | 250 | -220 | 30 | 0 | -20 |

# FIG.9

| MEASURE | EFFECT CHANGE | AREA CHANGE | STRUC-TURAL CHANGE |
|---|---|---|---|
| f11 | - | - | - |
| m12 | 1.0 | 0.25 | 0.1 |
| m11 | 0.9 | 0.85 | 0.82 |

# FIG.10

STRUCTURAL CHANGE
(EMPATHY)

RC2

AREA CHANGE
(EXECUTABILITY)

EFFECT CHANGE
(EFFECTIVENESS)

——— MEASURE CANDIDATE m11

------- MEASURE CANDIDATE m12

# FIG.11

START

S101
RECEIVE MEASURE EVALUATION
REQUEST

S102
ACQUIRE EXISTING MEASURE FLOW AND
M MEASURE CANDIDATE FLOWS

LOOP PROCESSING 1
NUMBER M OF MEASURE CANDIDATES

LOOP PROCESSING 2
NUMBER N OF INDEXES

S103
CALCULATE EVALUATION VALUE
FOR n-TH INDEX BETWEEN
EXISTING MEASURE AND
m-TH MEASURE CANDIDATE

END LOOP PROCESSING 2

END LOOP PROCESSING 1

S104
OUTPUT EVALUATION VALUES FOR
EACH INDEX BETWEEN EXISTING
MEASURE FLOW AND M MEASURE
CANDIDATE FLOWS

END

# FIG.12

EP 4 730 234 A1

## INTERNATIONAL SEARCH REPORT

| International application No. |
| --- |
| **PCT/JP2023/022487** |

| A. | CLASSIFICATION OF SUBJECT MATTER |
| --- | --- |

*G06Q 10/0633*(2023.01)i
FI:  G06Q10/0633

According to International Patent Classification (IPC) or to both national classification and IPC

| B. | FIELDS SEARCHED |
| --- | --- |

Minimum documentation searched (classification system followed by classification symbols)

G06Q10/0633

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2022-107998 A (HITACHI, LTD.) 25 July 2022 (2022-07-25)<br>entire text, all drawings | 1-18 |
| A | JP 2022-118681 A (TOSHIBA TEC KABUSHIKI KAISHA) 15 August 2022 (2022-08-15)<br>entire text, all drawings | 1-18 |
| A | JP 2017-068875 A (HITACHI, LTD.) 06 April 2017 (2017-04-06)<br>entire text, all drawings | 1-18 |
| A | JP 2021-117837 A (HITACHI, LTD.) 10 August 2021 (2021-08-10)<br>entire text, all drawings | 1-18 |
| A | WO 2022/054262 A1 (NIPPON TELEGRAPH AND TELEPHONE CORPORATION) 17 March 2022 (2022-03-17)<br>entire text, all drawings | 1-18 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **03 August 2023** | **15 August 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

| International application No. |
| --- |
| **PCT/JP2023/022487** |

| Patent document cited in search report | | | Publication date (day/month/year) | Patent family member(s) | | | Publication date (day/month/year) |
| --- | --- | --- | --- | --- | --- | --- | --- |
| JP | 2022-107998 | A | 25 July 2022 | WO | 2022/153624 | A1 | |
| JP | 2022-118681 | A | 15 August 2022 | (Family: none) | | | |
| JP | 2017-068875 | A | 06 April 2017 | (Family: none) | | | |
| JP | 2021-117837 | A | 10 August 2021 | (Family: none) | | | |
| WO | 2022/054262 | A1 | 17 March 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021117837 A